# EUROPEAN PATENT SPECIFICATION

(11) **EP 1 809 578 B1**
(45) Date of publication and mention of the grant of the patent: **02.06.2010**
(21) Application number: 05794607.1
(22) Date of filing: 19.10.2005
(51) Int. Cl.: C02F 11/04

(54) **METHOD AND INSTALLATION FOR PRODUCING BIOGAS WITH ANAEROBIC HYDROLYSIS**
VERFAHREN UND VORRICHTUNG ZUR PRODUKTION VON BIOGAS MIT ANAEROBER HYDROLYSE
METHODE ET INSTALLATION DE PRODUCTION DE BIOGAZ A L'AIDE DE L'HYDROLYSE ANAEROBiE

(30) Priority: 19.10.2004 DK 200401599
(43) Date of publication of application: 25.07.2007
(73) Proprietor: Bio-Circuit ApS, 8382 Hinnerup (DK)
(72) Inventor: JENSEN, Jan, DK-4000 Roskilde (DK); JENSEN, Preben, DK-8300 Odder (DK)
(74) Representative: Jakobsen, Gert Hoey
(86) International application number: PCT/DK2005/000677
(87) International publication number: WO 2006/042551

(56) References cited:
- EP-A- 0 566 056
- EP-A- 0 810 041
- DE-A1- 19 612 010

## Description

### FIELD OF THE INVENTION

The present invention relates to a method and a system for conversion of organic waste into biogas, i.e. a methane containing gas, with an improved efficiency and economy.

### BACKGROUND OF THE INVENTION

Typically, today's biogas producing facilities depend on supply of industrial waste containing fat to be economically feasible. Fat has a high energy to weight ratio, which makes it a useful input for biogas producing facilities. There is a high demand for industrial waste for this purpose, which has made it a rather expensive and limited resource. Thus, there is a need for a biogas producing facility that makes it possible to substitute industrial waste with other materials, e.g. other low-energy waste materials.

Conventionally, organic material from e.g. wastewater plants and livestock dung is processed in simple systems. Systems requiring addition of enzymes and/or chemicals has been developed, but suffers from high operational costs from the additives, hereby being economically unfavourable.

Although, digestion of excess wastewater sludge reduces the amount of final dewatered sludge, the final disposal of the sludge still has high costs. Also, many wastewater treatment plants have insufficient organic matter for de-nitrification.

In WO 2005/000748, the present inventors disclose a biogas producing facility wherein organic waste is subjected to a digesting step in a first reactor, and subsequently to an anaerobic hydrolysis step in an anaerobic tank, whereby the energy content of material that was not digested in the first reactor is made available for bacterial digestion and thus, the hydrolysed material is fed into a second reactor, or, is fed back into the first reactor for further bacterial conversion into biogas. Accordingly, the anaerobic hydrolysis step significantly increases the produced amount of biogas compared to a similar facility without the hydrolysis step. This is believed to be caused by the fact that performing a hydrolysis process on a biomass with a high content of volatile and easily digestible and reactive volatiles induces a tendency for constituents of organic matter to denature or condense during hydrolysis into derivatives of organic matter that cannot be digested in the reactor. Therefore such materials may advantageously be digested in a reactor before subjection to hydrolysis.

The document EP 0 810 041 (Ebara corporation) discloses an other conventional system for the conversion of organic waste into biogas.

### SUMMARY OF THE INVENTION

The present invention provides a method and a system with increased yield for economically feasible conversion of organic waste into biogas. The method according to the present invention comprises the consecutive steps of: i) digestion of the organic waste in a first reactor; ii) hydrolysis of the digested organic waste in an anaerobic hydrolysis tank; and iii) digestion of the hydrolyzed organic waste in a second reactor; prior to step iii) the evolved gases are removed from the anaerobic hydrolysis tank, by passing a gas through the headspace of the anaerobic hydrolysis tank.

### DETAILED DESCRIPTION OF THE INVENTION

It is an advantage of the present invention that a method and a system for conversion of organic waste into biogas is provided with an increased yield of biogas, without the addition of chemicals, enzymes or water, hereby providing an economically more feasible production of biogas.

Thus, according to the present invention, a method of producing biogas from organic waste is provided, comprising the consecutive steps of:
i) digestion of the organic waste in a first reactor;
ii) hydrolysis of the digested organic waste in an anaerobic hydrolysis tank; and
iii) digestion of the hydrolyzed organic waste in a second reactor;
wherein evolved gases are removed from the anaerobic hydrolysis tank.

In one aspect of the present invention the above-mentioned method may be performed in a biogas producing facility comprising a first reactor for holding organic waste for production of biogas by digestion and having an output for digested waste, and an anaerobic tank that is connected to the reactor output for anaerobic hydrolysis of the digested waste, and having an output for hydrolysed material that is connected to an input of a second reactor for adding hydrolyzed material to the content of the reactor and
wherein a gas is passed through the headspace of the anaerobic hydrolysis tank for removal of gases from the digested waste.

Organic waste to be converted to biogas in a method or a system according to the present invention may be any kind of waste or biomass, such as organic fertilizer, manure, semi liquid manure, livestock dung, animal remains, animal feed remains, bacterial material, household waste, industrial waste, industrial waste water, sludge, such as biological sludge from sewage treatment plants, etc., planting stock, such as corn, grass, dry grass, fresh or dry straw, straw contained in livestock dung, straw contained in deep-bedding, etc., fibres, silage, or mixtures thereof.

In the anaerobic hydrolysis tank, the hydrolysis of the digested waste according to the present invention is primarily provided by a temperature depende nt swelling and softening of the material. Also hydrolysis to a very small extent may take place by an enzymatic hydrolysis or a bacterial action by naturally occurring bacteria. En zymes produced by the bacteria in the first reactor as well as some bacteria may be transferred from the first reactor together with the digested waste to the hydrolysis tank. Due to the applied temperature, for example in the range above about 60 °C, the hydrolysis process is not favourable for bacterial growth, and also only a small part of the transferred enzymes may survive being able to function.

Increased temperature decreases the duration of the hydrolysis a nd enables an efficient hydrolysis without addition of bacteria, enzymes or chemicals. It is a further advantage of the present invention that no further chemicals are added to assist the anaerobic hydrolysis process.

During digestion of organic waste material in the reactor and during hydrolysis of digested waste in the anaerobic hydrolysis tank, various gases are produced; among these are hydrogen sulphide and ammonia/ammonium. Hydrogen sulphide originates from sulphate salts and proteins wherein amino acids may have some content of reduced sulphur. By digestion of biological substance, which takes place at neutral pH , the produced hydrogen sulphide will be present in the liquid where it is formed as well as in the produced biogas. Ammonia/ammonium is formed by digestion of urine and protein since urine has a high content of reduced nitrogen, and amino acids have an amino group.

In water at neutral pH gases, such as ammonia, hydrogen sulphide, and carbon dioxide are partly soluble and the corresponding equilibriums are establis hed:

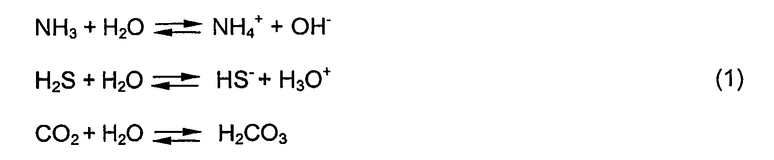

The amount of gases, that evaporates into the headspace of the reactor or hydrolysis tank depends on the temperature of the liquid, the pH value, and the partial pressure of these gases over the liquid. Thus, heating of the biological substances during the hydrolysis, leads to evaporation of a number of volatile compositions, such a s organic acids, carbon dioxide, ammonia and hydrogen sulphide.

The inventors have surprisingly found that by passing a gas through the headspace of the anaerobic hydrolysis tank, hereby removing the evaporated gasses from the headspace and enhancing a further evaporation of gasses from the digested waste, an improved and faster digestion is provided when the hydrolysed waste is digested in the second reactor. By passing a gas with a low partial pressure of e.g. H₂S and/or NH₃, the above-mentioned equilibriums between the gasses in solution and in gaseous state are driven to the left, whereby further gas, such as CO₂, H₂S and/or NH₃, is removed from the waste. Thus, the hydrolysed waste leaving the hydrolysis tank has a lower content of ammonia, sulphide, carbon dioxide and other volatiles that may inhibit the biogas forming bacteria in the second reactor. Accordingly, the digestion of the biomass organic matter into biogas will be enhanced both with relation to the amount of biogas generated and with relation to the rate of biogas production. Furthermore, due to the reduced content of gases, such as e.g. ammonia and sulphide, the temperature at which the biogas production takes place in the second reactor may be higher, without the gases adversely affects the naturally occurring bacteria. Therefore, the second reactor is very effective in converting hydrolysed organic matter to biogas compared to previously described biogas producing systems.

In a preferred embodiment of the invention, the evolved gases are removed by passing a gas through the headspace of the anaerobic hydrolysis tank.

In principle, for this purpose any gas, or mixture of gasses, with the appropriate partial pressures could be applied. However, in large scale plants to minimize corrosion of the tank as well as reduce large scale risks of explosions it is preferred to use a substantially non-oxygen containing gas, a low oxygen containing gas or mixture of gases, such as biogas, nitrogen gas (N₂) or similar gases, exhaust gas, or any other low or non-oxygen containing gas available. In one embodiment of the invention, the gas passed through the headspace is biogas output from the first reactor, or alternatively, in another embodiment of the invention, it is biogas output from the second reactor. In a preferred embodiment of the invention, the gas passed through the headspace is a combined biogas output from the first and the second reactor.

In another embodiment of the invention, the gas passed through the headspace may be a nitrogen gas (N₂), a non-oxygen containing gas, such as argon gas (Ar), a low oxygen containing gas, an exhaust gas, such as exhaust gas from a gas engine, gas boiler, petrol engine, diesel engine etc., or mixtures thereof, that optionally are recycled over the hydrolysis tank headspace, heated before and cooled after the headspace to remove evolved gases from the hydrolysis tank.

In small-scale systems, the gas may be constituted by atmospheric air. In relation to the volume of the headspace and the therein obtained contact between the atmospheric air and the waste being hydrolyzed, atmospheric air may in the context of the present invention be regarded as a low oxygen containing gas, since the gas is not mixed into the material so that the process stays anaerobic.

Furthermore, the gas may be continuously or discontinuously passed through the headspace of the anaerobic hydrolysis tank.

In one embodiment of the invention the removal of gasses from the hydrolysis tank is further enhanced by passing the gas through the hydrolysis tank at a higher rate than the corresponding rate of gas production from the waste being hydrolyzed. Thus, the partial pressures of the gasses, e.g. ammonia, are reduced, and additional amounts of e.g. ammonia and/or other volatiles will evaporate.

The inventors have furthermore found, that a heated gas enhances the removal of gasses from the hydrolysis tank, compared to a gas having ambient temperature. Accordingly, in a method according to the invention the gas to be passed through the headspace of the anaerobic hydrolysis tank may be heated in a heat exchanger before passing through the headspace of the hydrolysis tank. In a preferred embodiment of the invention, the gas is heated to a temperature of from about 15 °C to about 95 °C, preferably from about 50 °C to about 90 °C, or more preferably from about 75 °C to about 85 °C, in a heat exchanger, such as a counter current heat exchanger.

Alternatively, in another embodiment of the invention the gases evolved in the hydrolysis tank, may be removed from the headspace of the anaerobic hydrolysis tank through a flow path to downstream process equipment. The flow path to downstream process equipment may be provided by a communication between the headspace of the anaerobic hydrolysis tank and either the biogas outlet from the first reactor or the biogas outlet from the second reactor. However, the flow path to a downstream processing may alternatively be provided by a communication between the headspace of the anaerobic hydrolysis tank and the combined biogas outlets from the first reactor and the second reactor. Alternatively the flow path to a downstream processing may be provided by a pipe to a gas purification system, such as an odour cleaning system.

In a producing facility the temperature will typically be maintained with an accuracy of about +/- one to two degrees from the selected temperature. In some facilities the accuracy may even vary with as much as +/- three degrees from the selected temperature. Accordingly, if the temperature for a specific reactor or tank is selected to be 40 °C, the actually reactor temperature may be from about 39 °C to about 41 °C, such as from about 38 °C to about 42 °C or from about 37 °C to about 43 °C.

In the method according to the present invention, the digestion in step i) may be performed, in a first reactor, at a temperature of from about 10 °C to about 70 °C, preferably from about 20 °C to about 60 °C, more preferably from about 30 °C to about 55 °C, even more preferably from about 35 °C to about 50 °C, or at a temperature of 40 °C. Furthermore, the digestion of the organic waste in step i) may typically be performed for about 1 to about 50 days, preferably for about 5 to about 40 days, more preferably for about 15 to about 30 days or even more preferably for about 10 to about 20 days. The time required for the digestion in a first reactor depends on a variety of factors, such as the type of organic waste and the temperature. The digestion process for wastewater sludge is for example much faster than the digestion process for livestock dung containing straw. In a preferred embodiment of the invention, the digestion in step i) is performed at a temperature of from about 30 °C to about 55 °C for about 15 to about 30 days, preferably from about 35 °C to about 50 °C for about 15 to about 30 days.

In the method according to the present invention, the hydrolysis in step ii) may be performed, in an anaerobic hydrolysis tank, at a temperature of from about 55 °C to about 95 °C, preferably from about 65 °C to about 90 °C, more preferably from about 75 °C to about 85 °C, or even more preferably at a temperature of about 80 °C. As mentioned above, the actually reactor temperature may vary with for example +/- one to two degrees from the selected temperature, such as from about 78 °C to about 82 °C. Furthermore, the digestion of the organic waste in step ii) may typically be performed for about 0.25 to about 60 hours, preferably for about 4 to about 30 hours, more preferably for about 8 to about 20 hours, or even more preferably especially in large scale facilities for about 14 to about 16 hours. The below mentioned examples, are performed in laboratory scale, and accordingly the hydrolysis time required i.e. especially the time required to remove evolved gases from the hydrolyzed waste, in theses examples are shorter than the time required in a large scale facility. This difference mainly arise from the larger bulk and hereby a more slow and/or difficult release of the evolved gases to th e headspace of the tank. In a preferred embodiment of the invention, the hydrolysis in step ii) is performed at a temperature of from about 75 °C to about 85 °C, for about 8 to about 20 hours, preferably from about 75 °C to about 85 °C for about 14 to about 16 hours, or more preferably at a temperature of about 80 °C for about 14 to about 16 hours.

As mentioned above, due to the removal of evolved gases, the temperature in step iii) in a second reactor may be higher than normally expected for a reactor for digestion of organic waste. In the method according to the invention, the digestion in step iii) may be performed, in a second reactor, at a temperature of from about 15 °C to about 70 °C, preferably from about 35 °C to about 65 °C, more preferably from about 45 °C to about 60 °C, or even more preferably at a temperature of about 55 °C. As mentioned above, the actually reactor temperature may vary with for example +/- one to two degrees from the selected temperature, such as from about 53 °C to about 57 °C, when the selected temperature is about 55 °C. Furthermore, as described above, the rate of formation of biogas is also higher, and the digestion in step iii) may typically be performed for about 1 to about 50 days, preferably for about 15 to about 30 days and more preferably for about 10 to about 15 days. As mentioned for the first digestion step the time required for the digestion depends on a variety of factors, such as the type of organic waste and the temperature, in the second reactor the time required furthermore depends on how efficient the hydrolysis step, and especially the removal of gases during the hydrolysis, has been. As described previously, the rate of digestion in the second reactor, is increased when evolved gases, such as ammonia and hydrogen sulphide, are removed prior to the second digestion step. In a preferred embodiment of the invention, the digestion in step iii) is performed at a temperature of from about 45 °C to about 60 °C , for about 15 to about 30 days, preferably from about 55 °C for about 15 to about 30 days, or more preferably at a temperature of about 55 °C for about 10 to about 15 days.

In a livestock dung biogas producing facility, the gas produced typically has a high content of carbon dioxide, hydrogen sulphide and ammonia. It is a furth er object of this invention to clean the biogas produced in the system, hereby reducing th e content of especially hydrogen sulphide to avoid wear and damaging of gas motors, etc., which transforms the biogas into electricity and heat. Since the gas output from the hydrolysis tank has an increased temperature, the hydrogen sulphide and ammonia may be removed from the gas outlet by cooling in the above-mentioned counter current heat exchanger. By lowering the temperature, the evaporated hydrogen sulphide together with evaporated water and ammonia condenses, and may hereby be removed from the biogas. Depending on the composition of the organic materials that are processed, the amount of evaporated and thus removed ammonia varies. Where a larger amount of ammonia is present in the produced gas, it is furthermore possible to clean the produced biogas from significant amounts of carbon dioxide likewise by a condensation and a salt formation reaction. In the condensate, ammonium sulphide, ammonium carbonate and ammonium bicarbonate are formed whereby the above-mentioned equilibriums are driven to the right, which further increases the condensation of hydrogen sulphide, ammonia and carbon dioxide from the biogas.

In a preferred embodiment, the biogas producing facility further comprises a heat exchanger for heating the gas to be passed through the headspace of the anaerobic tank. Preferably, the gas that has been passed through the heads pace of the anaerobic hydrolysis tank is cooled in the heat exchanger so that condensable gases of the gases removed from the anaerobic hydrolysis tank content condense, the condensed water containing the removed gases. Among other things, this condensate is rich in ammonium carbonate, ammonium bicarbonate, and ammonium sulphide.

The inventors have surprisingly found, that the gasses formed in the hydrolysis tank thus may be applied to clean the biogas produced in the reactor tanks. When the biogas produced in one or both of the reactor tanks is used as the gas for removal of evaporated gas in the hydrolysis tank.

The biogas producing facility may further comprise a mixer in the anaerobic hydrolysis tank for continuously or discontinuously mixing the content of the tank.

In one embodiment of the method or the producing facility according to the invention, the first reactor also constitutes the second reactor.

Provision of anaerobic hydrolysis after digestion in the first reactor has the advantage that the amount of organic material to be processed in the anaerobic hydrolysis tank is kept at a minimum since the normally digestible part of the material has already been digested in the reactor. This reduces the required capacity of the anaerobic tank and related interconnecting systems thereby reducing investments and operational cost.

Preferably, the anaerobic hydrolysis in the anaerobic tank is performed at a pressure that is substantially equal to or higher than the saturation vapour pressure.

Surprisingly, it has been found that the output of the anaerobic hydrolysis substantially does not smell.

Preferably, the reactor is an anaerobic reactor due to its low operational cost.

In another embodiment of the invention, the biogas producing facility further comprises a separator that is connected to the first reactor output for selective separation of particles larger than a predetermined threshold size from the digested waste and having an output for the separated particles that is connected to the anaerobic tank for hydrolysis of the separated large particles.

Larger particles constitute most of the biological substance and thus, the useful biological substance is separated from the material that has been digested in the first reactor for further processing in the hydrolysis tank. This further reduces the required capacity of the anaerobic hydrolysis tank and related interconnecting systems, which in turn further reduces investments and cost. Alternatively, the hydrolysis step according to the present invention may be performed in at least one, such as two, three, four or five or more hydrolysis tanks operated in parallel. The smaller particles have a large content of biological dry matter that can not be digested, for example lign in-like substances, salts of phosphor, etc, which it is not desirable to feed into the hydrolysis tank. Thus, the dry matter subjected to subsequent hydrolysis has low phosphor content.

The separation efficiency may further be enhanced by adding precipitation agents or polymers whereby the particle size upstream the separation unit is increased leading to improved retention of solids for downstream hydrolysis.

For hydrolysis of sludge from wastewater treatment plants, the threshold size is preferably 1.0 mm, and more preferred 2.0 mm.

For hydrolysis of straw or similar material, the threshold size is preferably 0.2 cm, more preferred 0.5 cm, even more preferred 1.0 cm, still more preferred 1.5 cm, and most preferred 2.0 cm.

The separator may further comprise a dewatering device for dewatering of the separated particles.

In another embodiment of the invention, the biogas producing facility further comprises a second separator that is connected to the hydrolysis tank output for selective separation of particles larger than a second predetermined threshold size from the hydrolysed waste and having an output for the separated particles that is connected to the second reactor for digestion of the hydrolysed particles and an output for the reject water that is connected to a de-nitrification chamber of a wastewater treatment plant and / or to an anoxic fermentation chamber of a biological bio-P reduction chamber of a wastewater treatment plant.

Hydrolysis is preferably performed at a pressure that is substantially equal to or higher than the saturation vapour pressure. The pressure may be substantially equal to the ambient pressure, i.e. approximately 1 atmosphere, for provision of a simple and inexpensive hydrolysis system.

For some materials, performing the hydrolysis at higher pressures than ambient pressure, such as the saturation pressure at a temperature of 125 °C, 190 °C, etc, may optimise the efficiency and economics of the biogas producing facility. Increased temperature decreases the duration of the hydrolysis. For example, hydrolysis may be performed at a temperature in the range from 50 °C - 75 °C for 0.25 to 24 hours, or at a temperature in the range from 70 °C - 100 °C for 0.25 to 16 hours, e.g. for 4 to 10 hours, or at a temperature in the range from 100 °C - 125 °C for 0.25 to 8 hours, e.g. for 3 to 6 hours, or at a temperature in the range from 125 °C -150 °C for 0.25 to 6 hours, e.g. for 2 to 4 hours, or at a temperature in the range from 150 °C - 175 °C for 0.25 to 4 hours, e.g. for 1 to 2 hours, or at a temperature in the range from 175 °C - 200 °C for 0.25 to 2 hours, e.g. for 0.25 to 1 hours and at higher temperatures at even a shorter time. Due to the energy costs required to heat the hydrolysis tank, the preferred temperatures in step ii) according to the method of the present invention, are as described above, i.e., a temperature of e.g. about 75 °C to about 85°C for about 14 to about 16 hours.

The biogas producing facility may further comprise a partitioning device for partitioning of org anic waste and having an output for supplying the partitioned waste to the reactor.

The biogas producing facility according to the present invention has made it possible to substitute industrial waste with organic waste, such as corn, grass, dry grass, straw, silage, animal remains, etc. The straw may for example be fresh or dry straw or straw contained in livestock dung or in deep bedding. Thus, in a preferred embodiment, livestock dung mixed with straw is fed into the reactor. Straw has a dry matter content of 90 - 95 % and in spite of the fact that the fat content of straw is very low; it has a sig nificant energy content. The mixed dung and straw is digested in the reactor. After dig estion, remaining straw parts are separated in the separator and entered into the anaerobic tank for hydrolysis.

The hydrolysis of material after digestion in the first well functioning reactor together with a removal of evolved gases during the hydrolysis step increases the amount of produced gas by 20% to 80% or in some cases even more, depending on the organic waste processed, compared to the amount of gas produced in the first reactor without a subsequent anaerobic hydrolysis process. Typically, the amount of gas produced according to the present invention is increase by 25 - 50 %. Transportation of material by pumping using common biomass pumps requires that the dry matter content of the pumped material be kept below app. 15 % dry matter. At a larger cost, worm conveyors may be provided for pumping material with a dry matter content of up to app. 25 - 30 %. If the facility receives waste material with a high dry matter content, further waste material, such as straw, may not be added into the first reactor, but may instead be added to the content of the hydrolysis tank. Surprisingly, it has been found that feeding cut straws directly into the anaerobic hydrolysis tank results in a substantially homogenous mixture of straw and liquid in the tank, including a significantly reduced tendency for the straw to produce swim layer during downstream processing.

In an embodiment of the invention, gas produced in the hydrolysis tank is also provided to the first or second reactor or to the biogas handling and treatment system for further improvement of the biogas producing and treatment process. These gases are fed into the reactor or to the biogas handling and treatment system whereby the overall temperature in the biogas is increased. Hereby, it will be easier to maintain a constant and elevated pressure, since evaporated ammonia etc does not accumulate in the anaerobic tank including the tank headspace, but is output from the tank.

The amount of ammonia that may evaporate during this procedure may constitute until 50 % of the ammonia content in the biomass or even 70 % or more if the concentration in the biomass is high.

In a simple embodiment of the system, i.e. a system without a hydrolysis tank, the ammonia evaporation may take place in the reactor headspace. Thus, the biogas produced in the reactor headspace is cooled in a heat exchanger and recycled to the reactor headspace at a rate that is several times the rate of biogas production. The amount of ammonia evaporating de pends on the temperature in the digesting biomass. Thus, it is preferred to use high temperature digestion to enhance the ammonia evaporation, as for example 45°C digestion temperature or higher. The evaporation of ammonia makes it possible to increase the digester temperature without getting severe inhibition from ammonia and from hydrogen sulphide.

### EXAMPLES

In the examples below, hydrolysis duration and temperature are adapted to the small-scale laboratory experiments. In a full-scale facility the duration time ranges will be different due to the increased amounts of organic waste processed, for example, when there in the below examples are mentioned a hydrolysis duration of 8 hours, the corresponding hydrolysis duration in a full-scale facility may be around 12 to 16 hours. Furthermore, where the removal of evolved gases are performed, by passing a gas through the headspace of the hydrolysis tank; atmospheric air constitutes the gas for convenience. However, any gas with an appropriate partial pressure, as described in the description, may be applied. Where nothing else is mentioned in the below examples, the hydrolysis time applied is 8 hours at a temperature of 80 °C.

### Example 1

### Increased biogas production from semi liquid manure by removal of evolved gases

Experiments have been performed on semi liquid manure to investigate the effect of a hydrolysis tank with a communication between the headspace of the anaerobic hydrolysis tank and a biogas outlet from a reactor, compared to a conventional closed hydrolysis tank.

After the first digestion of the material, three experiments (A, B and C) were performed; the total biogas productions after the subsequent digestion steps are given below:

| Conditions | Biogas produced in m³ / 1000 Kg dry matter supplied. |
|---|---|
| A) control experiment, no hydrolysis; | 26, |
| B) hydrolysis in a closed hydrolysis tank; | 72, (176% increase compared to experiment A) |
| C) hydrolysis in a hydrolysis tank with a communication between the headspace and a biogas outlet from a reactor. | 107, (311% increase compared to experiment A) (49% increase compared to experiment B) |

It is noted that the removal of gases from the hydrolysis tank (C) has a surprisingly highly positive effect on the total biogas yield, both compared to a two-step digestion procedure without hydrolysis (311% increase compared to experiment A) as well as compared to a two-step digestion with an intermediate hydrolysis step (49% increase compared to experiment B).

### Example 2

### Increased biogas production from sludge by removal of evolved gases

Experiments have been performed on sludge from a sewage treatment plant, with respect to temperature of gas and hydrolysis time, to investigate the effect on the total biogas production. Experiments were performed with either atmospheric air with ambient temperature or with heated atmospheric air passing through the headspace of the hydrolysis tank.

After the first digestion of the sludge, four experiments (A control, B, C, and D) were performed; B, C and D where each repeated five times as addition experiments, the total biogas productions after the subsequent digestion steps are given below:

| Conditions | Biogas produced in m³ / 1000 Kg dry matter supplied. | | | | | Mean, m³ | % Increase compared to exp. A) |
|---|---|---|---|---|---|---|---|
| A) control experiment, no hydrolysis; | 67 | - | - | - | - | 67 | - |
| B) 4 hours hydrolysis, atmospheric air, ambient temperature; | 56 | 72 | 84 | 82 | 103 | 79 | 18% |
| C) 18 hours hydrolysis, atmospheric air, ambient temperature; | 70 | 88 | 99 | 104 | 109 | 94 | 40% |
| D) 8 hours hydrolysis, atmospheric air, heated; | 120 | 127 | 131 | 131 | 133 | 128 | 91 % |

It is noted that the removal of gases from the hydrolysis tank by passing a gas through the headspace of the hydrolysis tank, has a surprisingly positive effect on the total biogas yield compared to a two-step digestion procedure without hydrolysis (B and C compared to A). Furthermore, the heating of the gas (he re atmospheric air) prior to flushing the hydrolysis tank gives an even higher production of biogas (D compared to A, B, C).

### Example 3

### Increased biogas production from a mixture of semi liquid manure and industrial waste by removal of evolved gases

As it was seen in example 1, the removal of gases from the headspace by a communication between the headspace of the anaerobic hydrolysis tank and a biogas outlet from a reactor gave an increased yield. Experiments have furthermore been performed on a mixture of semi liquid manure and industrial waste, with regard to the influence of hydrolysis time and the time range wherein the communication between the headspace and a biogas outlet is provided.

After the first digestion of the material, four experiments (A, B, C and D) were performed; the total biogas productions after the subsequent digestion steps are given below:

| Conditions | Biogas produced in m³ / 1000 Kg dry matter supplied. |
|---|---|
| A) control experiment, no hydrolysis; | 153, |
| B) 2 hours hydrolysis in a hydrolysis tank with a communication between the headspace and a biogas outlet from a reactor for the first 30 min; | 164, (7% increase compared to experiment A) |
| C) 8 hours hydrolysis in a hydrolysis tank with a communication between the headspace and a biogas outlet from a reactor for the first 30 min; | 179, (17% increase compared to experiment A) (9% increase compared to experiment B) |
| D) 8 hours hydrolysis in a hydrolysis tank with a communication between the headspace and a biogas outlet from a reactor for the entire 8 hours; | 233, (52% increase compared to experiment A ) (30% increase compared to experiment C) |

The energy content of the applied material is higher than in the material used in example 1 and 2, experiment A shows that the a two-step digestion without a hydrolysis step gives a higher biogas yield than seen in example 1 or 2, but it is noted that it is still possible to increase the biogas yield further by the method according to the invention. Accordingly, the removal of gases from the hydrolysis tank (B, C, and D) has a surprisingly positive effect on the total biogas yield compared to the two-step digestion procedure without hydrolysis (7%, 17% and 52% increase compared to experiment A). Furthermore, It is noted that even a 30 min communication between the headspace of the hydrolysis tank and a biogas outlet gives an increased overall biogas yield. However, an increased hydrolysis time gives a further increased biogas production (9% compared to B), as well as a provided communication between the headspace of the hydrolysis tank and a biogas outlet for the full hydrolysis time gives a further increased biogas production (30% increase compared to C).

### Example 4

### Removal of gases from semi liquid manure during the hydrolysis step, measured as remaining ammonia/ammonium after the final digestion step.

Experiments have been performed on semi liquid manure, with respect to temperature of gas and hydrolysis time, to investigate to what extent a gas passed through the headspace of the hydrolysis tank removes gases, such as ammonia, from the organic waste, hereby influencing the total biogas production. As mentioned in the description, the digestion in a second reactor is surprisingly faster and more efficient, when evolved gases, such as ammonia, are removed during the hydrolysis step. Experiments were performed with either gas with ambient temperature or with heated gas passing through the headspace of the hydrolysis tank.

After the first digestion of the semi liquid manure, six experiments (A control, B, C, D, E and F) were performed; after the subsequent digestion step the amount of ammonia/ammonium remaining in the remaining organic waste was measured, the amounts are given below:

| Conditions | Amount ammonia/ammonium remaining, measured in the organic waste after the second digestion step, (kg / 1000 kg waste) |
|---|---|
| A) control experiment (two repetitions), no hydrolysis; | 3.3, 3.4, mean 3.35 kg |
| B) 4 hours hydrolysis, gas, ambient temperature; | 2.2, (34.3% removed compared to experiment A) |
| C) 8 hours hydrolysis, gas, ambient temperature; | 1.6, (52.2% removed compared to experiment A) |
| D) 2 hours hydrolysis, gas, heated; | 3.2, (4.4% removed compared to experiment A) |
| E) 4 hours hydrolysis, gas, heated; | 1.6, (52.2% removed compared to experiment A) |
| F) 8 hours hydrolysis, gas, heated; | 0.1, (97.0% removed compared to experiment A) |

It is noted that the method according to the present invention reduces the amount of ammonia/ammonium from the digested and hydrolyzed waste. The temperature of the gas applied and the hydrolysis time influences the removal of gases. For semi liquid manure with a high level of nitrogen containing material the optimal ammonia stripping conditions are in this example an 8 hours period with a heated gas passed through the headspace of the hydrolysis tank, where after there is nearly no ammonia left in the processed semi liquid manure.

### BRIEF DESCRIPTION OF THE DRAWINGS

- Fig. 1: schematically illustrates containers and their interconnections in a biog as producing facility according to the present invention,
- Fig. 2: schematically illustrates a biogas producing facility according to the present invention suited for waste having a low dry matter content,
- Fig. 3: schematically illustrates a biogas producing facility according to the present invention suited for waste having a high dry matter content,
- Fig. 4: schematically illustrates another biogas producing facility according to the present invention suited for waste having a high dry matter content, and
- Fig. 5: schematically illustrates the hydrolysis tank of a biogas producing facility according to the present invention.
- Fig. 6: schematically illustrates the hydrolysis tank of a biogas producing facility combined with headspace flushing according to the present invention

### DETAILED DESCRIPTION OF THE DRAWINGS

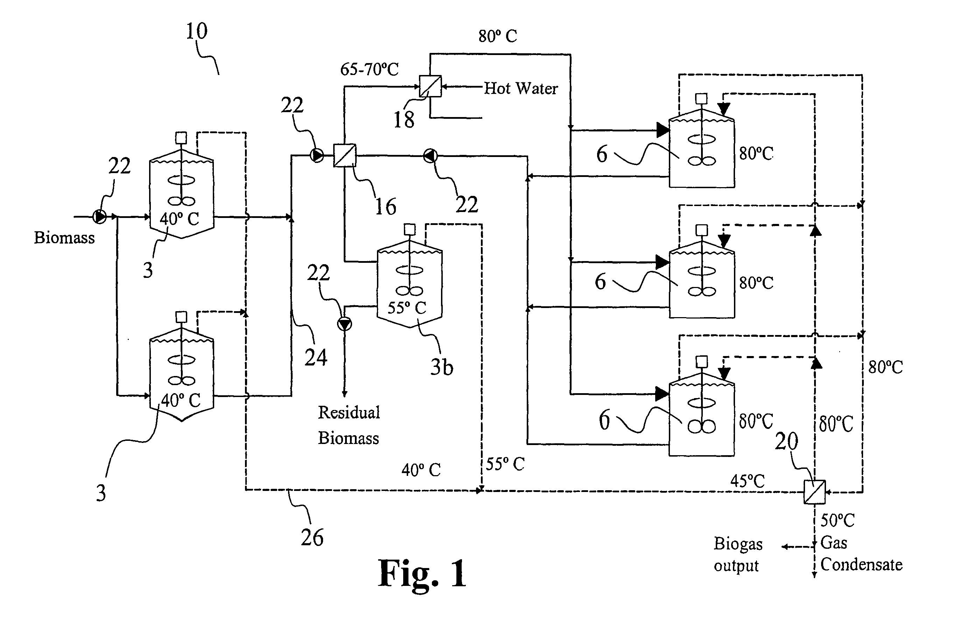

Fig. 1 schematically illustrates containers, i.e. reactors and hydrolysis tanks, a nd their interconnections in a biogas producing facility 10 according to the present invention. The optionally heat-treated biomass is fed into first reactors 3 operated in parallel for digestion of the biomass by bacteria for formation of biogas. Typically, the biomass is di gested for approximately 15 - 30 days depending on the reactor temperature. Typically, the reactor temperature ranges from 30 °C - 55 °C.

Upon digestion, the digested biomass is entered into a set of three anaerobic hydrolysis tanks 6 operated in parallel. A heat exchanger 16 is positioned between the outputs of the reactors 3 and the inputs of the hydrolysis tanks 6 for heating of the digested biomass. A further heat exchanger 18 is positioned downstream the heat exchanger 16 for further heating of the biomass, e.g. by hot water, e.g. pressurized hot water, before entrance into the anaerobic hydrolysis tanks 6. In the hydrolysis tanks, the digested biomass is typically hydrolysed at app. 80 °C for about 14 to about 16 hours. Upon hydrolysis the hydrolysed biomass is output to a second reactor 3b for further digestion. The output from the hydrolysis tanks 6 constitutes the counter flowing medium of the heat exchanger 16 so that the output from the hydrolysis tanks 6 is cooled before entrance into the second reactor 3b. Feeding pumps for pumping biomass are shown with reference numeral 22. The pipes 24 used for transportation of biomass between the containers 3, 3b, 6 are indicated with solid lines. Pipelines 26 for transportation of gas are indicated with dashed lines.

Gas formed during the hydrolysis is washed out from the headspace of the hydrolysis tanks 6 using biogas from the first and second reactors 3, 3b and transferred to the biogas handling and treatment system. Hereby, the biogas produced by the digestion in the reactors 3, 3b wash out evaporated water and gases from the hydrolysis tanks 6, and thus cleans the biomass in the hydrolysis tanks 6 for those gases as explained in the description. Further, the temperature of the gas in the system is increased so that the efficiency of the biological cleaning process or a similar process is increased, and the ammonia content of the biogas is increased so that the efficiency of binding hydrogen sulphide and other acidic gases is enhanced.

The heat exchanger 20 heats the biogas to be passed through the headspace of the anaerobic tanks 6. The washed out gas constitutes the counter current flow medium in the heat exchanger 20 whereby the gas from the headspace of the anaerobic hydrolysis tanks 6 is cooled in the heat exchanger 20 leading to condensation of condensable gases of the gases removed from the anaerobic hydrolysis tanks 6, the condensed water containing the removed gases. Among other things, this condensate is rich in ammonium carbonate, ammonium bicarbonate, and ammonium sulphide. Thus, as already mentioned previously, the gasses formed in the hydrolysis tank clean the biogas produced in the reactor tanks 3, 3b.

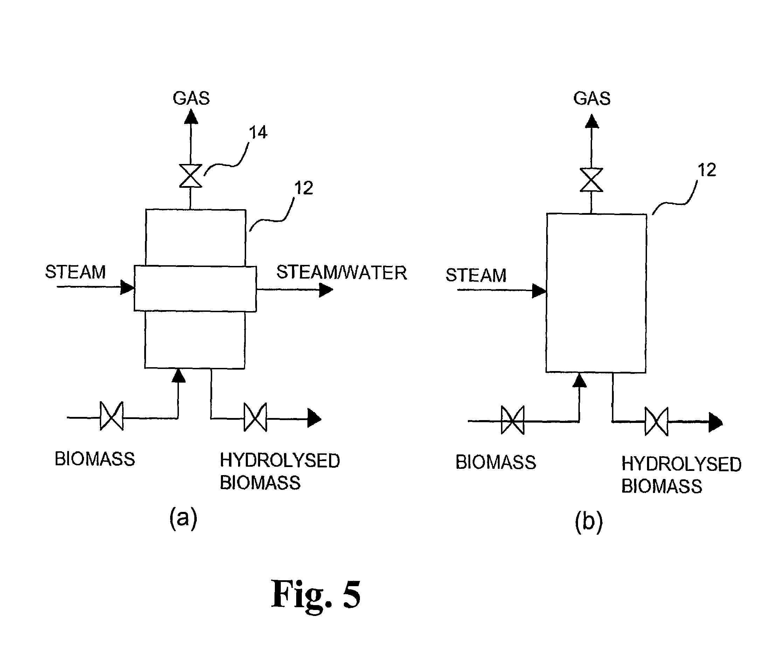

The anaerobic tanks 6 may be pressurized by steam either directly or via a mantle as is further explained below with reference to Fig. 5, or, an increased pressure may be generated by the feeding pump feeding material into the anaerobic hydrolysis tank 6.

It is an advantage of the biogas producing facility according to the invention that no further containers for chemical and/or biological processing are needed between the reactors and the hydrolysis tanks mentioned in the present disclosure whereby a simple producing facility is provided.

Likewise in the method according to the present invention, no further chemical and/or biological processing steps are required between the consecutive processing steps mentioned in the present disclosure.

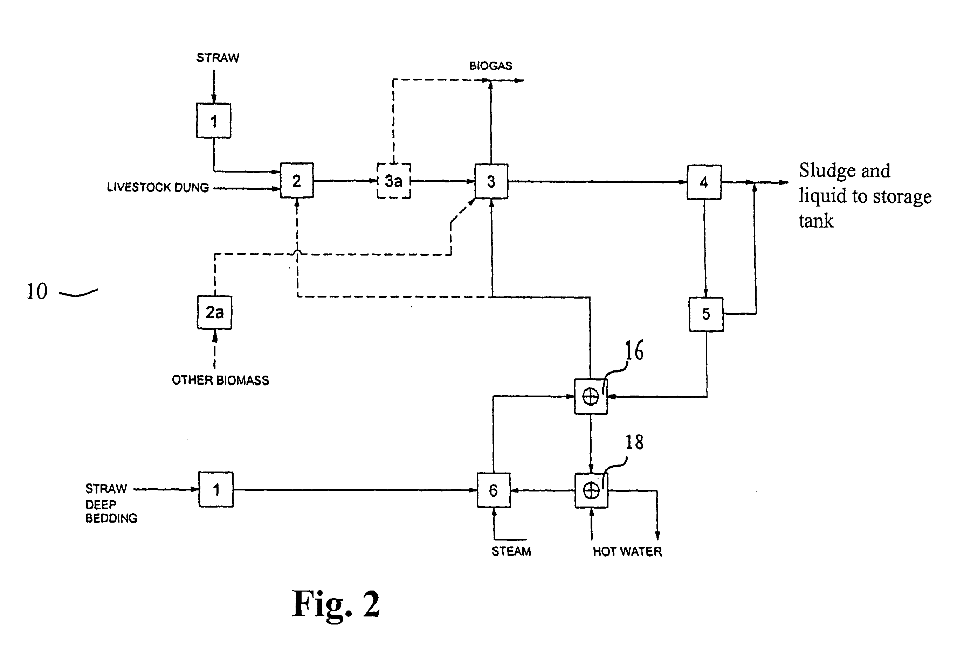

Fig. 2 schematically illustrates a biogas producing facility 10 for producing biogas from livestock dung mixed with organic waste, such as corn, grass, dry grass, fresh or dry straw, straw contained in livestock dung or in deep-bedding, silage, animal remains, etc. In the illustrated embodiment, the dung has low dry matter content so that a substantial amount of straw may be added to the dung. A partitioning device 1 cuts straw into straw parts having a mean length of approximately 5 to 10 cm. The cut straws and livestock dung are mixed in a tank 2, and the mixed matter is optionally heat treated in a tank 3a, typically at 70 - 75 °C, to kill unwanted bacteria. The optionally heat-treated matter is fed into a first reactor 3 to be digested by bacteria for formation of biogas. Typically, the matter is digested for approximately 15 - 30 days depending on the reactor temperature. Typically, the reactor temperature ranges from 30 °C - 55 °C. A separator 4 separates particles larger than 0.2 cm to 2 cm, and the separated particles may be de-watered in a second separator 5 whereby the dry matter content reaches 10 - 15 % dry matter. The separated matter is entered into the anaerobic hydrolysis tank 6 for anaerobic hydrolysis.

Optionally, the output from the separator 4 is entered into the anaerobic hydrolysis tank 6 through a heat exchanger 16. Then, the output from the hydrolysis tank constitutes the other medium of the heat exchanger 16 whereby the output from the hydrolysis tank is cooled before entrance into the first reactor 3, which constitutes the second reactor according to the invention.

Also optionally, the output from the separator 4 may be heated in a heat exchanger 18, e.g. by hot water, e.g. pressurized hot water, before entrance into the anaerobic hydrolysis tank 6.

Still optionally, organic waste, such as corn, grass, dry grass, fresh or dry straw, straw contained in livestock dung or in deep-bedding, silage, etc, may also be fed directly into the anaerobic hydrolysis tank 6, or, the organic waste may be mixed with at least some of the output from the first reactor 3 in a tank before entrance into the anaerobic hydrolysis tank 6. '

For example, cut straw may be fed directly into the anaerobic hydrolysis tank 6. Surprisingly, it has been found this causes a substantially homogenous mixture of straw and liquid to be formed in the tank 6.

The anaerobic tank 6 may be pressurized by steam either directly or via a mantle as is further explained below with reference to Fig. 5, or, an increased pressure may be generated by the feeding pump feeding material into the anaerobic hydrolysis tank 6.

The hydrolysed matter is dissolved in liquid or takes the form of small particles.

Another biological substance 2a may be supplied to the facility 10, such as industrial waste, sorted household garbage, etc. This other biological substance is fed directly into the first reactor tank 3, and therefore it does not influence the other parts of the system.

Besides the output from the hydrolysis tank may be passed to a separator and / or dewatering unit to separate the liquid from the solids. The liquid has an elevated content of volatile organic matter, and is suitable for de-nitrification and for biological Bio-P phosphorous reduction at wastewater treatment plants.

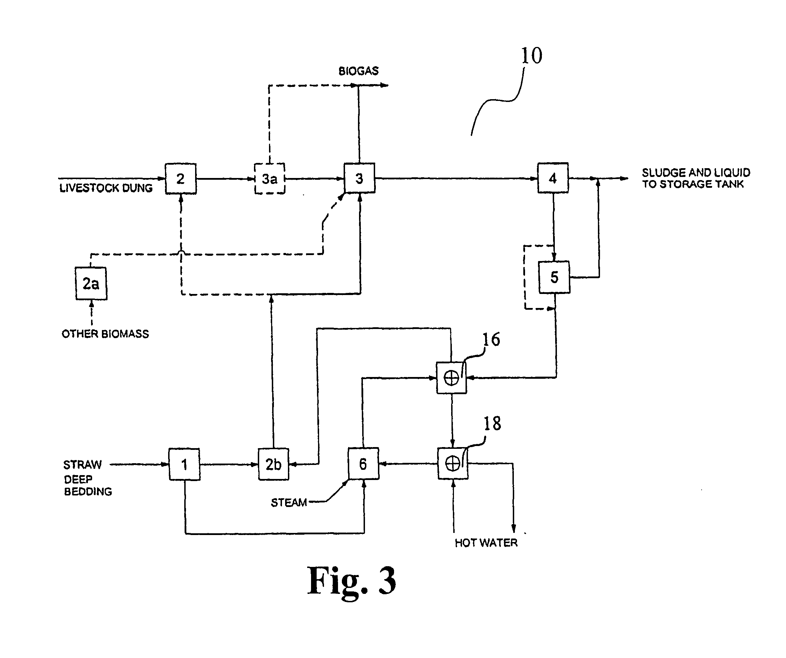

Fig. 3 schematically illustrates a biogas producing facility 10 for producing biogas from livestock dung mixed with straw. The mixed dung and straw has high dry matter content. A partitioning device 1 cuts straw into straw parts having a mean length of approximately 5 to 10 cm. The cut straws and hydrolysed material are mixed in a tank 2b, and the mixed matter is fed into a first reactor 3 to be digested by bacteria for formation of biogas. Alternatively or additionally, the cut straws may be entered directly into the anaerobic tank 6. Surprisingly, it has been found that a substantially homogenous mixture of straw and liquid is formed in the tank 6.

Livestock dung is mixed in 2 and optionally heat-treated in 3a. The optionally heat-treated matter is also fed into the first reactor 3 to be digested by bacteria for formation of biogas. Typically, the matter is digested for approximately 15 - 30 days depending on the reactor temperature. Typically, the reactor temperature ranges from 30 °C - 55 °C. A separator 4 separates particles larger than 0.2 cm to 2 cm and the separated particles may be dewatered in a second separator 5 whereby the dry matter content reaches 10 - 15 % dry matter. The separated matter is entered into the hydrolysis tank 6 for hydrolysis.

Optionally, the output from the separator 4 is entered into the anaerobic hydrolysis tank 6 through a heat exchanger 16. Then, the output from the hydrolysis tank constitutes the other medium of the heat exchanger 16 whereby the output from the hydrolysis tank is cooled before entrance into the first reactor 3, which constitutes the second reactor according to the invention.

Also optionally, the output from the separator 4 may be heated in a heat exchanger 18, e.g. by hot water, e.g. pressurized hot water, before entrance into the anaerobic hydrolysis tank 6.

The anaerobic tank 6 may be pressurized by steam either directly or via a mantle as is further explained below with reference to Fig. 5, or, an increased pressure may be generated by the feeding pump feeding material into the anaerobic hydrolysis tank 6.

Gasses evolved in the headspace of the hydrolysis tank may be removed by passing a gas through the headspace of the anaerobic hydrolysis tank, e.g. by passing biogas from the reactor(s) through the headspace as shown in Fig. 1. Alternatively, gasses may be removed from the headspace of the anaerobic hydrolysis tank through a flow path to downstream process equipment as explained previously.

The hydrolysed matter is dissolved in the liquid or takes the form of small particles.

For livestock dung with a high content of dry mater, it may be unnecessary to de-water the separated particles. The dashed line indicates a bypass of the second separator 5.

Another biological substance 2a may be supplied to the facility 10, such as industrial waste, sorted household garbage, etc. This other biological substance is fed directly into the first reactor tank 3, and therefore it does not influence the other parts of the system.

Besides the output from the hydrolysis tank may be passed to a separator and / or dewatering unit to separate the liquid from the solids. The liquid has an elevated content of volatile organic matter, and is suitable for de-nitrification and for biological Bio-P phosphorous reduction at wastewater treatment plants.

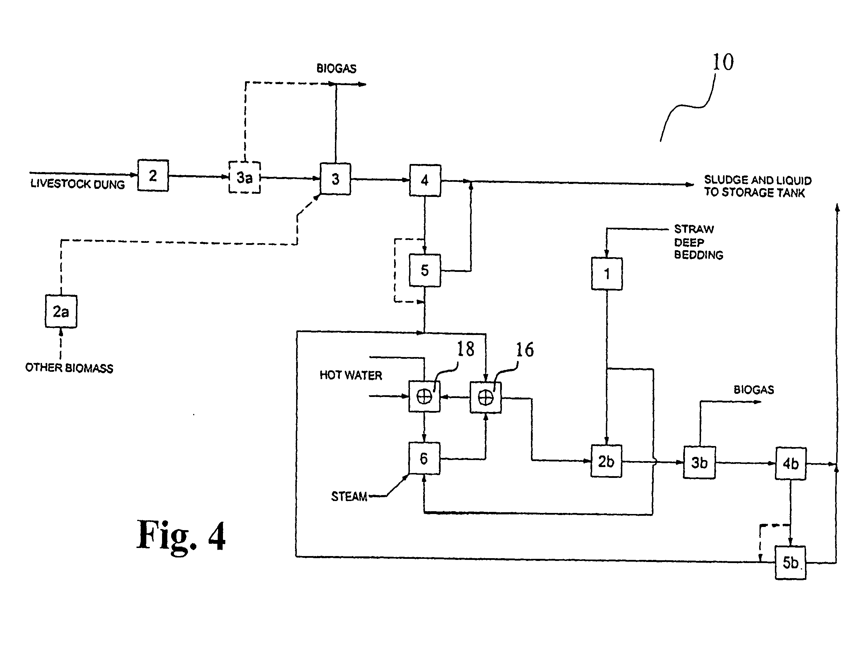

Fig. 4 schematically illustrates another biogas producing facility 10 for producing biogas from livestock dung mixed with straw. The mixed dung and straw has high dry matter content. Livestock d ung is mixed in 2 and optionally heat-treated in 3a at a temperature of about 70 - 75 °C. The optionally heat-treated matter is fed into a first re actor 3 to be digested by bacteria for formation of biogas. Typically, the matter is digested for approximately 15 - 30 days depending on the reactor temperature. Typically, the reactor temperature ranges from 30 °C - 55 °C. A separator 4 separates particles larger than 0.2 cm to 2 cm and the separated particles may be de-watered in a second separator 5 whereby the dry matter content reaches 10 - 15 % dry matter. The separated matter is entered into the hydrolysis tank 6 for hydrolysis.

The anaerobic tank 6 may be pressurized by steam either directly or via a mantle as is further explained below with reference to Fig. 5, or, an increased pressure may be generated by the feeding pump feeding material into the anaerobic hyd rolysis tank 6.

Gasses evolved in the headspace of the hydrolysis tank may be removed by passing a gas through the headspace of the anaerobic hydrolysis tank, e.g. by passing biogas from the reactor(s) through the headspace as shown in Fig. 1. Alternatively, gasses may be removed from the headspace of the anaerobic hydrolysis tank through a flow path to downstream process equipment as explained previously.

The hydrolysed matter is dissolved in the liquid or takes the form of small particles.

A partitioning device 1 cuts straw into straw parts having a mean length of approximately 5 to 10 cm. The cut straws and hydrolysed material from tank 6 are mixed in a tank 2b.

The mixture is digested in a second reactor 3b. A separator 4b separates particles larger than 0.2 cm to 2 cm, and the separated particles may be de-watered in another separator 5b whereby the dry matter content reaches 10 - 15 % dry matter. The separated matter is entered into the hydrolysis tank 6 for hydrolysis together with the output from the first reactor 3.

Alternatively or additionally, the cut straws may be entered directly into the anaerobic tank 6. Surprisingly, it has been found that a substantially homogenous mixture of straw and liquid is formed in the tank 6.

The hydrolysed matter is dissolved in the liquid or takes the form of small particles.

Optionally, the output from the separator 4 and the output from se parator 4b are entered into the anaerobic hydrolysis tank 6 through a heat exchanger 16. Then, the output from the hydrolysis tank constitutes the other medium of the heat exchanger 16 whereby the output from the hydrolysis tank 6 is cooled before entrance into the first reactor 3.

Also optionally, the output from the separator 4 may be heated in a heat exchanger 18, e.g. by hot water, e.g. pressurized hot water, before entrance into the anaerobic hydrolysis tank 6.

For livestock dung with a high content of dry mater, it may be unnecessary to de-water the separated particles. A bypass of the second separator 5b is indicated by the dashed line.

Another biological substance 2a may be supplied to the facility 10, such as industrial waste, sorted household garbage, etc. This other biological substance is fed directly into the first reactor tank 3, and therefore does not influence the other parts of the system.

Besides the output from the hydrolysis tank may be passed to a separator and / or dewatering unit to separate the liquid from the solids. The liquid has an elevated content of volatile organic matter, and is suitable for de-nitrification and for biological Bio-P phosphorous reduction at wastewater treatment plants.

Fig. 5 schematically illustrates the hydrolysis tank of an embodiment of the invention
wherein the gas formed during the hydrolysis is output to the reactor or the biogas handling and treatment system. Hereby, the evolved gases during hydrolysis are removed from the hydrolysis tank, prior to the second digestion step, performed in a second reactor, the biogas produced by the digestion may furthermore be cleaned as explained above, and the temperature of the gas in the system is increased so that the efficiency of the biological cleaning process or a similar process may be increased.

In the illustrated embodiment, biological material to be hydrolysed is input to the hydrolysis tank 12. Depending on the desired hydrolysis temperature, the anaerobic tank is heated by steam injected directly into the tank as illustrated in Fig. 5b or by heating a mantle or pipes surrounding the tank as illustrated in Fig. 5a. Alternatively or additionally, the input entered into the anaerobic hydrolysis tank 12 through a heat exchanger (not shown). Then, the output from the hydrolysis tank constitutes the other medium of the heat exchanger whereby the output from the hydrolysis tank is cooled before entrance into the reactor. Also optionally, the input to the tank 12 may be further heated in a second heat exchanger (not shown), e.g. by hot water, e.g. pressurized hot water, before entrance into the anaerobic hydrolysis tank 12.

During temperature increase in the tank, the hydrolysis gas output valve 14 is open so that gas formed by the hydrolysis process in the headspace above the biological material communicates with gas formed by digestion in the reactor (not shown). When the biological liquid has reached the decided temperature, communication with the biogas produced in the reactor may be maintained at least for at predetermined period, such as for the entire hyd rolysis period. If the pressure is to be increased, the valve 14 is closed, and when the desired pressure is reached, the valve and the supply of heat is controlled to maintain a substantially constant pressure in the tank. During hydrolysis under pressure, CO₂ and other gasses are formed by auto oxidation of organic material and dissolved in the liquid and in bacteria in the liquid. Upon pressure release, the pressure of dissolved gasses contained in the bacteria will disrupt the bacteria membranes and thus, destroy the bacteria.

Having finished hydrolysis, the headspace valve 14 may again be opened to avoid low pressure (vacuum) in the anaerobic tank. The temperature in the anaerobic tank may be decreased by release of steam to the reactor gas or the gas handling system, or, cooling may be effected utilizing heat exchange or heat recovery.

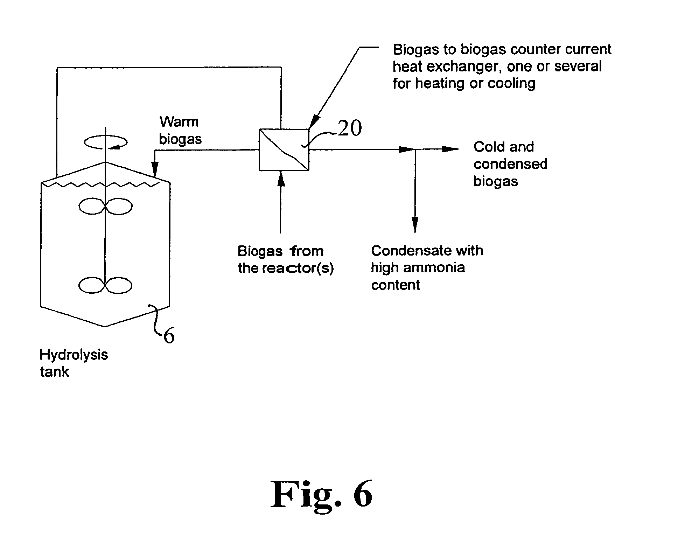

Fig. 6 schematically illustrates the hydrolysis tank of an embodiment of the invention
wherein the gas formed during the hydrolysis is washed out from the headspace of the hydrolysis tank using biogas and transferred to the biogas handling and treatment system. Hereby, the biogas produced by the digestion in the reactor wash out evaporated water and gases from the hydrolysis tank, and thus cleans the biomass in the hydrolysis tank for those gases as explained in the description. Further, the temperature of the gas in the system is increased so that the efficiency of the biological cleaning process or a similar process may be increased. Still further, the ammonia content of the biogas is increased so that the efficiency of binding hydrogen sulphide and other acidic gases is enhanced. The wash out may advantageously be utilized both in facilities wherein a second reactor is the same reactor as a first reactor, i. e. the hydrolysed biomass is re-circulated to the first reactor, and wherein the first and second reactors are different reactors.

The gas used to wash the headspace of the hydrolysis tank may be biogas from the biogas producing reactors, or exhaust gas from gas fire or gas engine, or air, or may be an unspecified recycled gas where the content of ammonia and hydrogen sulphide gas is condensed or bound by chemicals in a next step. The gas temperature may be lower than the temperature of the biomass in the headspace, but preferably the gas temperature is at about the same level as the biomass in the hydrolysis tank or at a hig her temperature.

Gas produced by the hydrolysis contains ammonia, hydrogen sulphid e, carbon dioxide, Volatile Fatty Acids (VFA), evaporated water, etc. The gas that has been passed through the headspace of the anaerobic hydrolysis tank is cooled in a heat exchanger 20 so that the condensable gases of the gases removed from the anaerobic hydrolysis tank content condense, the condensed water containing the removed gases.

In the embodiments illustrated in Figs. 2-4, the separators 4, 4b separate particles larger than a threshold value that is set in accordance with the type of mate rial digested in the reactor. For example, for hydrolysis of sludge from wastewater treatment plants, the threshold size is in the range from approximately 1.0 mm to approximately 2.0 mm, and for hydrolysis of fibre containing material, such as straw, the threshold size is in the range from approximately 0.2 cm to approximately 2.0 cm. The smaller particles have a high content of substances that cannot be microbially digested and a high content of salts of phosphor and nitrogen that desirably should not participate in the hydrolysis.

The separator may operate by sedimentation. However, sedimentation is not efficient in separating phosphor so lamella separators or vibrator screens etc may be preferred.

The output of the separator constitutes a liquid particle fraction of approximately 15 - 30 volume % of the separator input and contains approximately 20 - 50 % of the dry matter of the separator input and has a dry matter content of approximately 8 - 15 %.

If necessary, the second separators 5, 5b, increase the dry matter content to in the order of 10 - 15 % depending on whether the biogas producing facility is intended for livestock dung with a low dry matter content, or for livestock dung with high dry matter content. The separator 5, 5b may be a centrifuge or a screw press, etc.

The output of the separator 5, 5b constitutes a liquid particle fraction of 60 - 70 volume % of the separator input and contains 70 - 80 % of the dry matter of the separator input and has a dry matter content of 12 -15 %.

Laboratory experiments with wastewater treatment plant biological excess sludge show that biogas production using anaerobic digestion and subsequent anaerobic hydrolysis provides an enhancement of the biogas production by 50 to 70 % compared to the production by similar anaerobic digestion without anaerobic hyd rolysis. Similar experiments with animal manure or animal manure with added straw show that biogas production using anaerobic digestion and subsequent anaerobic hydrolysis provides an enhancement of the biogas production by 20 to 80 % compared to the production by similar anaerobic digestion without anaerobic hydrolysis. Naturally, the dry matter reduction corresponds to the biogas production.

Laboratory experiments with animal manure shows, that by evaporating ammonia and hydrogen sulphide gas and other gases using a headspace gas wash out with another gas, hydrogen sulphide and other substances from the biomass in the hydrolysis tank, provides an enhancement of the biogas production by further 20 to 80 % compared to the production by similar treatment without washing the gas in the hydrolysis tank headspace with another gas. The efficiency is the same for added biomass as straw, grass etc and results in a similar enhancement of the total biogas production. Thus the combination of hydrolysis of biomass and headspace wash out of certain gases produced during the hydrolysis process mutually enhance the conversion of biomass to biogas even further than hydrolysis does without headspace washout, also on animal manure, also on sludge from waste water treatment plants, also on added biomass as for example wet or dry straw, hay, grass, rapeseed hay and any other added co-substrate and biomass, and besides the conversion velocity from organic matter to biogas is enhanced.

At the same time the evaporated gases, among other things, ammonia and hydrogen sulphide is reclaimed as a condensate with high nitrogen fertilizer content e.g. several times higher than animal manure, wastewater etc. Thus, transportation costs, storage costs etc are low. Besides the fertilizer value is high.

A simple embodiment of the ammonia evaporation system may include a gas circuit over the headspace of the reactor. The biogas is cooled and condensed, and afterwards heated counter current the gas from the reactor. The gas has low vapour content and aids the evaporation of among other things, ammonia and water fro m the reactor surface. As the gas circuit recycles several times the gross gas production amount, a high amount of the ammonia may leave the reactor with the gas circuit, and may be condensed, thus allowing a higher digester temperature which again enhances the ammonia evaporation to the gas phase, and which will condense in the gas circuit.

## Claims

1. A method of producing biogas from organic waste comprising the consecutive steps of:
i) digestion of the organic waste in a first reactor;
ii) hydrolysis of the digested organic waste in an anaerobic hydrolysis tank; and
iii) digestion of the hydrolyzed organic waste in a second reactor;
wherein evolved gases are removed from the anaerobic hydrolysis tank by passing a gas through the headspace of the anaerobic hydrolysis tank.

2. A method according to claim 1, wherein the organic waste is organic fertilizer, manure, semi liquid manure, livestock dung, animal remains, animal feed remains, bacterial material, household waste, industrial waste, industrial waste water, sludge, corn, grass, dry grass, fresh or dry straw, straw contained in livestock dung, straw contained in deep-bedding, fibres, silage, or mixtures thereof.

3. A method according to any of claims 1 and 2, wherein the gas passed through the headspace is biogas output from the first reactor.

4. A method according to any of claims 1 and 2, wherein the gas passed through the headspace is biogas output from the second reactor.

5. A method according to any of the preceding claims, wherein the gas passed through the headspace is a combined biogas output from the first and the second reactor.

6. A method according to any of claims 1 and 2, wherein the gas passed through the headspace is a nitrogen gas (N₂), a non-oxygen containing gas, a low oxygen containing gas, an exhaust gas or a mixture thereof.

7. A method according to any of the preceding claims, wherein the digestion in step i) is performed at a temperature of from 10°C to 70°C, such as from 20 °C to 60 °C, from 30 °C to 55 °C, from 35 °C to 50 °C, or at a temperature of 40 °C.

8. A method according to any of the preceding claims, wherein the hydrolysis in step ii) is performed at a temperature of from 55 °C to 95 °C, such as from 65 °C to 90 °C, from 75 °C to 85 °C, or at a temperature of 80 °C.

9. A method according to any of the preceding claims, wherein the digestion in step iii) is performed at a temperature of from 15 °C to 70°C, such as from 35 °C to 65 °C, from 45 °C to 60 °C, or at a temperature of 55 °C.

10. A method according to any of the preceding claims, wherein the digestion in step i) is performed for 1 to 50 days, such as for 5 to 40 days, for 15 to 30 days or for 10 to 20 days.

11. A method according to any of the preceding claims, wherein the hydrolysis in step ii) is performed for 0.25 to 60 hours, such as for 4 to 30 hours, for 8 to 20 hours, or for 14 to 16 hours.

12. A method according to any of the preceding claims, wherein the digestion in step iii) is performed for 1 to 50 days, such as for 15 to 30 days or for 10 to 15 days.

13. A method according to any of claims 7 and 10, wherein the digestion in step i) is performed at a temperature of from 30 °C to 55 °C for 15 to 30 days, such as at a temperature of 35 °C to 50 °C for 15 to 30 days.

14. A method according to any of claims 8 and 11, wherein the hydrolysis in step ii) is performed at a temperature of from 75 °C to 85 °C, for 8 to 20 hours, such as from 75 °C to 85 °C for 14 to 16 hours, or at a temperature of 80 °C for 14 to 16 hours.

15. A method according to any of claims 9 and 12, wherein the digestion in step iii) is performed at a temperature of from 45 °C to 60 °C, for 15 to 30 days, such as of 55 °C for 15 to 30 days, or at a temperature of 55°C for 10 to 15 days.

16. A method according to claim 1, wherein the gas to be passed through the headspace of the anaerobic hydrolysis tank is heated in a heat exchanger.

17. A method according to claim 20, wherein the gas is heated to a temperature of from 15 °C to 95 °C, such as, e.g. preferably from 50°C to 90 °C, or more preferably from 75 °C to 85 °C, in a heat exchanger.

18. A method according to claim 17, wherein the gas, that has been passed through the headspace of the anaerobic hydrolysis tank, Is cooled in the heat exchanger so that condensable gases of the gases removed from the anaerobic hydrolysis tank content condense, the condensed water containing the removed gases.

19. A method according to claim 1, wherein the first reactor in step i) also constitutes the second reactor.

20. A biogas producing facility (10) comprising
a first reactor (3) for holding organic waste for production of biogas by digestion and having an output for digested waste, and
an anaerobic tank (3b) that is connected to the first reactor output for anaerobic hydrolysis of the digested waste and having an output for hydrolysed material that is connected to an Input of a second reactor for adding hydrolysed material to the content of the second reactor (6), wherein the headspace of the anaerobic hydrolysis tank further having an input and an output for a gas to be passed through the headspace of the anaerobic hydrolysis tank for removal of gases from the hydrolyzed waste.

21. A biogas producing facility (10) according to claim 20, wherein the gas input of the headspace is connected to a biogas output from the first reactor.

22. A biogas producing facility (10) according to claim 20, wherein the gas input of the headspace is connected to a biogas output from the second reactor.

23. A biogas producing facility (10) according to any of claims 20-22, wherein the gas input of the headspace is connected to a combined biogas output from the first and the second reactor.

24. A biogas producing facility (10) according to claim 20. wherein the gas input of the headspace is an inlet for nitrogen gas (N₂), a non-oxygen containing gas, a low oxygen containing gas, an exhaust gas or a mixture thereof.

25. A biogas producing facility (10) according to any of claims 20-24, further comprising a heat exchanger for heating the gas to be passed through the headspace of the anaerobic hydrolysis tank.

26. A biogas producing facility (10) according to claim 25, wherein the gas output of the headspace of the tank, is connected to the heat exchanger (20) so that condensable gases of the gases removed from the anaerobic hydrolysis tank content condense, the condensed water containing the removed gases.

27. A biogas producing facility (10) according to any of claims 20-26, further comprising a mixer in the anaerobic hydrolysis tank for continuously or discontinuously mixing the content of the tank.

28. A biogas producing facility (10) according to any of claims 20-27, further comprising a first separator that is connected to the first reactor output for selective separation of particles larger than a predetermined first threshold size from the digested waste and having an output for the separated large particles, and wherein the anaerobic tank is connected to the first separator output for anaerobic hydrolysis of the separated particles.

29. A biogas producing facility (90) according to any of claims 20-28, wherein the first reactor also constitutes the second reactor.

30. A biogas producing facility (10) according to any of claims 20-29, further comprising a second separator that is connected to the hydrolysis tank output for selective separation of particles larger than a second predetermined threshold size from the hydrolysed waste and having an output for the separated particles that is connected to the second reactor for digestion of the hydrolysed particles and an output for the reject water.

31. A biogas producing facility (10) according to claim 30, wherein the output for the reject water is connected to a de-nitrification chamber of a wastewater treatment plant.

32. A biogas producing facility (10) according to claim 30, wherein the output for the reject water is connected to an anoxic fermentation chamber of a biological bio-P reduction chamber of a wastewater treatment plant.

33. A biogas producing facility (10) according to any of claims 20-32, further comprising a gas circuit connected to the headspace of at least one of the first and second reactor having a heat exchanger for cooling and condensation of recycled biogas, for increased evaporation of ammonia in the reactor.

## Patentansprüche

1. Verfahren zur Herstellung von Biogas aus organischem Abfall, das die folgenden aufeinanderfolgenden Schritte umfasst:
i) Aufschluss des organischen Abfalls in einem ersten Reaktor;
ii) Hydrolyse des aufgeschlossenen organischen Abfalls in einem anaeroben Hydrolysetank; und
iii) Aufschluss des hydrolysierten organischen Abfalls in einem zweiten Reaktor;
wobei entwickelnde Gase aus dem anaeroben Hydrolysetank entfernt werden, indem man ein Gas durch den Kopfraum des anaeroben Hydrolysetanks leitet.

2. Verfahren gemäß Anspruch 1, wobei es sich bei dem organischen Abfall um organischen Dünger, Mist, Gülle, Viehdung, tierische Überreste, Tierfutterreste, bakterielles Material, Hausmüll, Industriemüll, Industrieabwasser, Schlamm, Getreide, Gras, trockenes Gras, frisches oder trockenes Stroh, in Viehdung enthaltenes Stroh, in tiefer Einstreu enthaltenes Stroh, Fasern, Silage oder Gemische davon handelt.

3. Verfahren gemäß einem der Ansprüche 1 und 2, wobei das durch den Kopfraum geleitete Gas aus dem ersten Reaktor entnommenes Biogas ist.

4. Verfahren gemäß einem der Ansprüche 1 und 2, wobei das durch den Kopfraum geleitete Gas aus dem zweiten Reaktor entnommenes Biogas ist.

5. Verfahren gemäß einem der vorstehenden Ansprüche, wobei das durch den Kopfraum geleitete Gas ein kombiniertes Biogas ist, das aus dem ersten und dem zweiten Reaktor entnommen ist.

6. Verfahren gemäß einem der Ansprüche 1 und 2, wobei das durch den Kopfraum geleitete Gas ein Stickstoffgas (N₂), ein nichtsauerstoffhaltiges Gas, ein sauerstoffarmes Gas, ein Abgas oder ein Gemisch davon ist.

7. Verfahren gemäß einem der vorstehenden Ansprüche, wobei der Aufschluss in Schritt i) bei einer Temperatur von 10 °C bis 70 °C, wie zum Beispiel von 20 °C bis 60 °C, von 30 °C bis 55 °C, von 35 °C bis 50 °C oder bei einer Temperatur von 40 °C durchgeführt wird.

8. Verfahren gemäß einem der vorstehenden Ansprüche, wobei die Hydrolyse in Schritt ii) bei einer Temperatur von 55 °C bis 95 °C, wie 65 °C bis 90 °C, 75 °C bis 85 °C oder bei einer Temperatur von 80 °C durchgeführt wird.

9. Verfahren gemäß einem der vorstehenden Ansprüche, wobei der Aufschluss in Schritt iii) bei einer Temperatur von 15 °C bis 70 °C, wie zum Beispiel von 35 °C bis 65 °C, von 45 °C bis 60 °C oder bei einer Temperatur von 55 °C durchgeführt wird.

10. Verfahren gemäß einem der vorstehenden Ansprüche, wobei der Aufschluss in Schritt i) 1 bis 50 Tage lang, wie zum Beispiel 5 bis 40 Tage lang, 15 bis 30 Tage lang oder 10 bis 20 Tage lang durchgeführt wird.

11. Verfahren gemäß einem der vorstehenden Ansprüche, wobei die Hydrolyse in Schritt ii) 0,25 bis 60 Stunden lang, wie zum Beispiel 4 bis 30 Stunden lang, 8 bis 20 Stunden lang oder 14 bis 16 Stunden lang durchgeführt wird.

12. Verfahren gemäß einem der vorstehenden Ansprüche, wobei der Aufschluss in Schritt iii) 1 bis 50 Tage lang, wie zum Beispiel 15 bis 30 Tage lang oder 10 bis 15 Tage lang durchgeführt wird.

13. Verfahren gemäß einem der Ansprüche 7 und 10, wobei der Aufschluss in Schritt i) bei einer Temperatur von 30 °C bis 55 °C während 15 bis 30 Tagen, wie zum Beispiel bei einer Temperatur von 35 °C bis 50 °C während 15 bis 30 Tagen, durchgeführt wird.

14. Verfahren gemäß einem der Ansprüche 8 und 11, wobei die Hydrolyse in Schritt ii) bei einer Temperatur von 75 °C bis 85 °C während 8 bis 20 Stunden, wie zum Beispiel bei einer Temperatur von 75 °C bis 85 °C während 14 bis 16 Stunden oder bei einer Temperatur von 80 °C während 14 bis 16 Stunden durchgeführt wird.

15. Verfahren gemäß einem der Ansprüche 9 und 12, wobei der Aufschluss in Schritt iii) bei einer Temperatur von 45 °C bis 60 °C während 15 bis 30 Tagen, wie zum Beispiel bei 55 °C während 15 bis 30 Tagen oder bei einer Temperatur von 55 °C während 10 bis 15 Tagen durchgeführt wird.

16. Verfahren gemäß Anspruch 1, wobei das durch den Kopfraum des anaeroben Hydrolysetanks zu leitende Gas in einem Wärmetauscher erhitzt wird.

17. Verfahren gemäß Anspruch 16, wobei das Gas in einem Wärmetauscher auf eine Temperatur von 15 °C bis 95 °C, wie zum Beispiel vorzugsweise von 50 °C bis 90 °C oder besonders bevorzugt von 75 °C bis 85 °C erhitzt wird.

18. Verfahren gemäß Anspruch 17, wobei das Gas, das durch den Kopfraum des anaeroben Hydrolysetanks geleitet wurde, in dem Wärmetauscher abgekühlt wird, so dass kondensierbare Gase der aus dem Inhalt des anaeroben Hydrolysetanks entfernten Gase kondensieren, wobei das kondensierte Wasser die entfernten Gase enthält.

19. Verfahren gemäß Anspruch 1, wobei der erste Reaktor in Schritt i) auch den zweiten Reaktor darstellt.

20. Biogasproduktionsanlage (10), umfassend:
einen ersten Reaktor (3) zur Aufnahme von organischem Abfall für die Produktion von Biogas durch Aufschluss mit einem Auslass für aufgeschlossenen Abfall; und
einen anaeroben Tank (3b), der mit dem Auslass des ersten Reaktors verbunden ist, für die anaerobe Hydrolyse des aufgeschlossenen Abfalls, einen Auslass für hydrolysiertes Material aufweisend, der mit einem Einlass eines zweiten Reaktors verbunden ist, um hydrolysiertes Material zu dem Inhalt des zweiten Reaktors (6) hinzuzufügen, wobei der Kopfraum des anaeroben Hydrolysetanks weiterhin einen Einlass und einen Auslass für ein Gas aufweist, das durch den Kopfraum des anaeroben Hydrolysetanks geleitet werden soll, um Gase aus dem hydrolysierten Abfall zu entfernen.

21. Biogasproduktionsanlage (10) gemäß Anspruch 20, wobei der Gaseinlass des Kopfraums mit einem Biogasauslass aus dem ersten Reaktor verbunden ist.

22. Biogasproduktionsanlage (10) gemäß Anspruch 20, wobei der Gaseinlass des Kopfraums mit einem Biogasauslass aus dem zweiten Reaktor verbunden ist.

23. Biogasproduktionsanlage (10) gemäß einem der Ansprüche 20 bis 22, wobei der Gaseinlass des Kopfraums mit einem kombinierten Biogasauslass aus dem ersten und dem zweiten Reaktor verbunden ist.

24. Biogasproduktionsanlage (10) gemäß Anspruch 20, wobei der Gaseinlass des Kopfraums ein Einlass für Stickstoffgas (N₂), ein nichtsauerstoffhaltiges Gas, ein sauerstoffarmes Gas, ein Abgas oder ein Gemisch davon ist.

25. Biogasproduktionsanlage (10) gemäß einem der Ansprüche 20 bis 24, die weiterhin einen Wärmetauscher umfasst, um das durch den Kopfraum des anaeroben Hydrolysetanks zu leitende Gas zu erhitzen.

26. Biogasproduktionsanlage (10) gemäß Anspruch 25, wobei der Gasauslass des Kopfraums des Tanks mit dem Wärmetauscher (20) verbunden ist, so dass kondensierbare Gase der aus dem Inhalt des anaeroben Hydrolysetanks entfernten Gase kondensieren, wobei das kondensierte Wasser die entfernten Gase enthält.

27. Biogasproduktionsanlage (10) gemäß einem der Ansprüche 20 bis 26, die weiterhin einen Mischer in dem anaeroben Hydrolysetank umfasst, um den Inhalt des Tanks kontinuierlich oder diskontinuierlich zu mischen.

28. Biogasproduktionsanlage (10) gemäß einem der Ansprüche 20 bis 27, die weiterhin einen ersten Separator umfasst, der zur selektiven Abtrennung von Teilchen, die größer als eine vorbestimmte erste Schwellengröße sind, aus dem aufgeschlossenen Abfall mit dem Auslass des ersten Reaktors verbunden ist und einen Auslass für die abgetrennten großen Teilchen aufweist, wobei der anaerobe Tank zur anaeroben Hydrolyse der abgetrennten Teilchen mit dem Auslass des ersten Separators verbunden ist.

29. Biogasproduktionsanlage (10) gemäß einem der Ansprüche 20 bis 28, wobei der erste Reaktor auch den zweiten Reaktor darstellt.

30. Biogasproduktionsanlage (10) gemäß einem der Ansprüche 20 bis 29, die weiterhin einen zweiten Separator umfasst, der zur selektiven Abtrennung von Teilchen, die größer als eine vorbestimmte zweite Schwellengröße sind, aus dem hydrolysierten Abfall mit dem Auslass des Hydrolysetanks verbunden ist und einen Auslass für die abgetrennten Teilchen, der zum Aufschluss der hydrolysierten Teilchen mit dem zweiten Reaktor verbunden ist, sowie einen Auslass für das Abwasser aufweist.

31. Biogasproduktionsanlage (10) gemäß Anspruch 30, wobei der Auslass für das Abwasser mit einer Denitrifikationskammer einer Abwasseraufbereitungsanlage verbunden ist.

32. Biogasproduktionsanlage (10) gemäß Anspruch 30, wobei der Auslass für das Abwasser mit einer anoxischen Fermentationskammer einer biologischen Phosphorreduktionskammer einer Abwasseraufbereitungsanlage verbunden ist.

33. Biogasproduktionsanlage (10) gemäß einem der Ansprüche 20 bis 32, die weiterhin einen Gaskreislauf umfasst, der mit dem Kopfraum des ersten und/oder zweiten Reaktors verbunden ist und einen Wärmetauscher zum Abkühlen und Kondensieren von recyceltem Biogas zur erhöhten Verdampfung von Ammoniak in dem Reaktor aufweist.

## Revendications

1. Un procédé de production de biogaz à partir de déchets organiques comprenant les étapes successives de:
i) digestion des déchets organiques dans un premier réacteur;
ii) hydrolyse des déchets organiques digérés dans une cuve d'hydrolyse anaérobique; et
iii) digestion des déchets organiques hydrolysés dans un second réacteur;
procédé dans lequel les gaz émis sont retirés de la cuve d'hydrolyse anaérobique par passage d'un gaz dans l'espace de tête de la cuve d'hydrolyse anaérobique.

2. Un procédé selon la revendication 1. dans lequel les déchets organiques consistent en engrais organiques, fumiers, fumiers semi liquides, fumiers animal, restes d'animaux, restes d'aliments pour animaux, matériaux bactériens, déchets ménagers, déchets industriels, effluents aqueux industriels, boues, blés, maïs, herbes, fanes, pailles fraîches ou sèches, pailles contenues dans du fumier animal, pailles contenues dans des litières, fibres, produits d'ensilage ou leurs mélanges.

3. Un procédé selon une quelconque des revendications 1 et 2, dans lequel le gaz passé dans l'espace de tête est du biogaz produit dans le premier réacteur.

4. Un procédé selon une quelconque des revendications 1 et 2, dans lequel le gaz passé dans l'espace de tête est du biogaz produit dans le second réacteur.

5. Un procédé selon une quelconque des revendications précédentes, dans lequel le gaz passé dans l'espace de tête est un mélange de biogaz produit dans les premiers et second réacteurs.

6. Un procédé selon une quelconque des revendications 1 et 2, dans lequel le gaz passé dans l'espace de tête est de l'azote gazeux (N₂), un gaz ne contenant pas d'oxygène, un gaz contenant une faible teneur en oxygène, un gaz effluant ou un de leurs mélanges.

7. Un procédé selon une quelconque des revendications précédentes, dans lequel la digestion dans l'étape i) est effectuée à une température comprise entre 10°C et 70°C, par exemple comprise entre 20°C et 60°C, entre_30°C et 55°C, entre 35°C et 50°C ou à une température de 40°C.

8. Un procédé selon une quelconque des revendications précédentes, dans lequel l'hydrolyse dans l'étape ii) est effectuée à une température comprise entre 55°C et 95°C, par exemple comprise entre 65°C et 90°C, entre 75°C et 85°C ou à une température de 80°C.

9. Un procédé selon une quelconque des revendications précédentes, dans lequel la digestion dans l'étape iii) est effectuée à une température comprise entre 15°C et 70°C, par exemple comprise entre 35°C et 65°C, entre 45°C et 60°C ou à une température de 55°C.

10. Un procédé selon une quelconque des revendications précédentes, dans lequel la digestion dans l'étape i) est effectuée sur une période de 1 à 50 jours, par exemple durant 5 à 40 jours, durant 15 à 30 jours ou durant 10 à 20 jours.

11. Un procédé selon une quelconque des revendications précédentes, dans lequel l'hydrolyse dans l'étape ii) est effectuée sur une période de 0,25 à 60 heures, par exemple de 4 à 30 heures, de 8 à 20 heures ou de 14 à 16 heures.

12. Un procédé selon une quelconque des revendications précédentes, dans lequel la digestion dans l'étape iii) est effectuée sur une période de 1 à 50 jours, par exemple de 15 à 30 jours ou de 10 à 15 jours.

13. Un procédé selon une quelconque des revendications 7 et 10, dans lequel la digestion dans l'étape i) est effectuée à une température comprise entre 30°C et 55°C pendant une période de 15 à 30 jours, par exemple à une température comprise entre 35°C et 50 °C pendant une période de 15 à 30 jours.

14. Un procédé selon une quelconque des revendications 8 et 11, dans lequel l'hydrolyse dans l'étape ii) est effectuée à une température comprise entre 75°C et 85°C sur une période de 8 à 20 heures, par exemple comprise entre 75°C et 85°C sur une période de 14 à 16 heures ou de 80°C sur une période de 14 à 16 heures.

15. Un procédé selon une quelconque des revendications 9 et 12, dans lequel la digestion dans l'étape iii) est effectuée à une température comprise entre 45°C et 60°C, sur une période de 15 to 30 jours, par exemple de 55°C sur une période de 15 à 30 jours, ou à une température de 55°C sur une période de 10 à 15 jours.

16. Un procédé selon la revendication 1, dans lequel le gaz destiné à passer dans l'espace de tête de la cuve d'hydrolyse anaérobique est chauffé dans un échangeur de chaleur.

17. Un procédé selon la revendication 20, dans lequel le gaz est chauffé dans un échangeur de chaleur à une température comprise entre 15°C et 95°C, telle que, p. ex., de préférence entre 50°C et 90 °C, ou plus préférablement entre 75°C et 85°C,.

18. Un procédé selon la revendication 17, dans lequel le gaz, qui est passé dans l'espace de tête de la cuve d'hydrolyse anaérobique, est refroidi dans un échangeur de chaleur de façon que les gaz condensables contenus dans le gaz retiré de la cuve d'hydrolyse anaérobique se condensent, l'eau condensée contenant les gaz retirés.

19. Un procédé selon la revendication 1, dans lequel le premier réacteur dans l'étape i) forme également le second réacteur.

20. Une installation (10) produisant du biogaz comprenant un premier réacteur (3) pour contenir des déchets organiques destinés à la production de biogaz par digestion et comportant une sortie pour les déchets digérés,
et une cuve anaérobique (3b) qui est relié à l'orifice de sortie des déchets digérés du premier réacteur d'hydrolyse anaérobique et comportant un orifice de sortie du matériau hydrolyse qui est relié à l'orifice d'entrée du second réacteur pour l'addition du matériau hydrolyse au contenu du second réacteur (6), dans lequel l'espace de tête de la cuve d'hydrolyse anaérobique comporte en outre un orifice d'entrée et un orifice de sortie pour le gaz passé dans l'espace de tête de la cuve d'hydrolyse anaérobique pour enlever des déchets hydrolysés les gaz.

21. Une installation (10) produisant du biogaz selon la revendication 20, dans laquelle l'orifice d'entrée du gaz dans l'espace de tête est relié à un orifice de sortie du biogaz du premier réacteur.

22. Une installation (10) produisant du biogaz selon la revendication 20, dans laquelle l'orifice d'entrée du gaz dans l'espace de tête est relié à un orifice de sortie du biogaz du second réacteur.

23. Une installation (10) produisant du biogaz selon une quelconque des revendications 20-22, dans lequel l'orifice d'entrée du gaz dans l'espace de tête est relié à un orifice de sortie du mélange du biogaz produit dans les premier et second réacteurs.

24. Une installation (10) produisant du biogaz selon la revendication 20, dans laquelle l'orifice d'entrée du gaz dans l'espace de tête est un orifice d'entrée pour l'azote gazeux (N₂), un gaz ne contenant pas d'oxygène, un gaz contenant une fable teneur en oxygène, un gaz effluant ou un de leurs mélanges.

25. Une installation (10) produisant du biogaz selon une quelconque des revendications 20-24, comprenant en outre un échangeur de chaleur pour chauffer le gaz destiné à être passé dans l'espace de tête de la cuve d'hydrolyse anaérobique.

26. Une installation (10) produisant du biogaz selon la revendication 25, dans laquelle l'orifice de sortie du gaz de l'espace de tête de la cuve, est relié à l'échangeur de chaleur (20) de façon que les gaz condensables contenus dans le gaz retiré de la cuve d'hydrolyse anaérobique se condensent, l'eau condensée contenant les gaz retirés

27. Une installation (10) produisant du biogaz selon une quelconque des revendications 20-26, comprenant en outre dans la cuve d'hydrolyse anaérobique des moyens d'agitation pour mélanger en continu ou en discontinu le contenu de la cuve.

28. Une installation (10) produisant du biogaz selon une quelconque des revendications 20-27, comprenant en outre un premier séparateur qui est relié à l'orifice de sortie du premier réacteur pour une séparation sélective des particules dont la dimension est supérieure à un premier seuil de dimension prédéterminé des déchets digérés et comportant un orifice de sortie pour les particules de grandes dimensions séparées, et dans laquelle la cuve anaérobique est reliée à l' orifice de sortie du premier séparateur d'hydrolyse anaérobique des particules séparées.

29. Une installation (10) produisant du biogaz selon une quelconque des revendications 20-28, dans laquelle le premier réacteur forme également le second réacteur.

30. Une installation (10) produisant du biogaz selon une quelconque des revendications 20-29, comprenant en outre un second séparateur qui est relié à l'orifice de sortie du premier réacteur pour une séparation sélective des particules dont la dimension est supérieure à un premier seuil de dimensions prédéterminé des déchets hydrolysés et comportant un orifice de sortie pour les particules séparées qui est relié à l' orifice de sortie du second réacteur pour la digestion des particules hydrolysées et un orifice de sortie pour l'effluent aqueux.

31. Une installation (10) produisant du biogaz selon la revendication 30, dans laquelle l'orifice de sortie pour l'effluent aqueux est relié à une chambre de-nitrification d'une installation de traitement des eaux usées.

32. Une installation (10) produisant du biogaz selon la revendication 30, dans laquelle l'orifice de sortie de l'effluent aqueux est relié à une chambre de fermentation anoxique d'une chambre de réduction bio-P biologique d'une installation de traitement des eaux usées.

33. Une installation (10) produisant du biogaz selon une quelconque des revendications 20-32, comprenant en outré un circuit de gaz relié à l'espace de tête d'au moins un des premier et second réacteurs comportant un échangeur de chaleur pour le refroidissement et la condensation du biogaz recyclé pour augmenter l'évaporation d'ammoniac dans le réacteur.
